# EUROPÄISCHE PATENTSCHRIFT

(11) **EP 0 779 079 B1**
(45) Veröffentlichungstag und Bekanntmachung des Hinweises auf die Patenterteilung: **31.03.2004**
(21) Anmeldenummer: 96118563.4
(22) Anmeldetag: 20.11.1996
(51) Int. Cl.: A61N 1/05

(54) **Einzel-Elektrodensonde für Zweikammer-Herzschrittmachersysteme, insbesondere für DDD-Herzschrittmachersysteme**
Single electrode lead for double-chamber cardiac stimulators, especially for DD cardiac stimulators
Sonde à électrode unique pour stimulateurs cardiaques double chambre, en particulier pour stimulateurs cardiaques de type DD

(30) Priorität: 15.12.1995 DE 19546941
(43) Veröffentlichungstag der Anmeldung: 18.06.1997
(73) Patentinhaber: BIOTRONIK Mess- und Therapiegeräte GmbH & Co Ingenieurbüro Berlin, 12359 Berlin (DE)
(72) Erfinder: Böhm, Mark Diran, 10829 Berlin (DE); Bolz, Armin, Dr., 91054 Buckenhof (DE); Thong, Tran, Dr., Portland, Oregon 97229 (US)
(74) Vertreter: Hübner, Gerd, Dipl.-Phys.

(56) Entgegenhaltungen:
- EP-A- 0 656 218
- WO-A-80/02801
- GB-A- 2 116 047
- US-A- 3 596 662
- US-A- 4 154 247
- US-A- 4 567 901

## Beschreibung

Die vorliegende Erfindung betrifft eine Einzel-Elektrodensonde - also eine sogenannte "Single Lead" - für Zweikammer-Herzschrittmachersysteme, insbesondere für DDD-Herzschrittmachersysteme, zum Einführen in das Herz mit den im Oberbegriff des Anspruches 1 angegebenen Merkmalen. In diesem Zusammenhang ist anzumerken, daß das eigentliche Schrittmachergerät einerseits und die Elektrodensonde andererseits eines Herzschrittmachersystems prinzipiell als getrennte Komponenten aufgefaßt werden. Um hier größtmögliche Kombinationsvielfalt zu gewährleisten, werden üblicherweise genormte Steckverbindungen zur elektrischen und mechanischen Verbindung zwischen Schrittmachergerät einerseits und Elektrodensonde andererseits verwendet. Ferner ist darauf hinzuweisen, daß solche Elektrodensonden auch für Defibrillatoren zum Einsatz kommen können.

Zu der dieser Erfindung zugrundeliegenden Problematik ist auszuführen, daß neuere Erkenntnisse der Herzschrittmachertechnik bei einschlägigen pathologischen Befunden die Anwendung von sogenannten DDD-Herzschrittmachersystemen empfehlen. Diese Spezifikation "DDD" (= Dual pacing/Dual sensing/Demand + Triggered) bedeutet, daß einerseits eine Stimulation ("Pacing") in beiden Herzkammern vorgenommen wird und daß andererseits noch vorhandene Eigenaktionen des Sinus- bzw. AV-Knotens des Herzens sowohl im Atrium, als auch im Ventrikel erfaßt werden ("Sensing"). Zu diesem Zweck sind bereits sogenannte "Double-Lead"-DDD-Schrittmachersysteme bekannt. Deren Hauptnachteil ist es jedoch, daß zwei getrennte Elektrodensonden in das Herz implantiert und dort positioniert werden müssen, nämlich eine üblicherweise in J-Form ausgebildete Elektrode im Atrium und eine weitere Elektrode im Ventrikel des Herzens. Die atriale J-Elektrode bringt einen hohen Implantationsaufwand mit sich, der von vielen Ärzten gescheut wird. Außerdem spricht eine erhöhte Gefahr von Thrombosen und infolgedessen von Embolien aufgrund der Thrombogenität der Elektrodensondenmaterialien gegen die Implantation einer Vielzahl von Elektrodensonden.

Einen aus Praxisgründen bisher akzeptierten Kompromiß stellen VDD-(= Ventricular pacing/Dual sensing/Demand + Triggered-) Herzschrittmachersysteme mit einer Einzel-Elektrodensonde dar. Deren Konstruktion umfaßt eine Einzelsonde, bei der eine ventrikuläre Elektrode an der Sondenspitze und zwei atriale Ringelektroden in entsprechender Entfernung von der Ventrikel-Elektrode am Sondenkörper angeordnet sind. Die atrialen Ringelektroden werden dazu verwendet, das atriale Potential zu erfassen, wodurch die ventrikuläre Stimulation nach einer geeigneten atrioventrikulären Verzögerung getriggert wird. Die ventrikuläre Stimulation kann bipolar erfolgen. Diese Art von Schrittmachersystem arbeitet zufriedenstellend bei Patienten mit stabiler Vorhofaktivität, guten atrialen Wahrnehmungseigenschaften und normaler Sinusknotenfunktion.

Grundsätzlich ist es zwar denkbar, die vorstehend erörterten Einzel-Elektrodensonden auch für DDD-Herzschrittmachersysteme einzusetzen. Jedoch stellt hier ein Problem dar, daß die atrialen Ringelektroden nicht wandständig plaziert, sondern frei im Blutstrom schwimmen. Diese sogenannten "flottierenden" Elektroden im Atrium führen zu erheblichen Einschränkungen der Stimulationseigenschaften, da meist sehr hohe Stimulationsamplituden benutzt werden müssen. Der Hauptgrund hierfür ist die fehlende Wandständigkeit der flottierenden Elektroden. Die hohen Stimulationsamplituden bringen den Nachteil eines hohen Energieverbrauches und häufiger Phrenikus-Stimulation mit sich, weswegen solche Einzel-Elektroden üblicherweise nur bei VDD-Herzschrittmachersystemen eingesetzt werden.

Ein weiterer Ansatz für eine Einzel-Elektrodensonde zur Verwendung in einem DDD-Herzschrittmachersystem ist aus dem Artikel von J. Hirschberg et al "New Dual Chamber Single Lead System" aus der Fachzeitschrift "PACE", November 1994, S. 1870 bis 1872 bekannt. Bei dieser Sonde handelt es sich um eine modifizierte bipolare Standard-Elektrode, deren Kopf im Atrium aktiv befestigt wird. Die ventrikuläre Elektrode ist als Ringelektrode ausgeführt. Zur Implantation wird nun die atriale Spitze mittels eines üblichen Führungsdrahtes - ein sogenannter "Mandrin" - an der Innenseite der rechten Außenwand des Vorhofes befestigt. Anschließend wird der Führungsdraht einige Zentimeter zurückgezogen und die Sonde mit ihrem Mittenbereich in das rechte Ventrikel vorgeschoben. Durch nochmaliges Einführen des Führungsdrahtes in die Sonde wird die dabei gebildete Sondenschlaufe, die die Ringelektrode trägt, in optimalem Kontakt mit der rechten Ventrikelwand gebracht. Anschließend wird der Führungsdraht herausgezogen, wobei eine ausreichende Sondenlänge innerhalb des rechten Ventrikels verbleibt, um eine Schlaufe innerhalb des Ventrikels zu bilden. Dabei tritt die Sonde zweimal durch die Tricuspidal-Klappe des Herzens hindurch. Eine ähnliche Einzel-Elektrodensonde ist auch aus der Druckschrift WO-A-8 002 801 zu entnehmen. Diese bekannte Struktur offenbart alle Merkmale des Oberbegriffs des Anspruchs 1.

Problematisch bei dieser vorbekannten Einzel-Elektrodensonde ist einerseits die Tatsache, daß die Elektrodenspitze im Atrium aktiv verankert werden muß, damit überhaupt das nachfolgende Einführen der Sonde in das Ventrikel in Form einer Schlaufe vonstatten gehen kann. Auch die Schlaufenbildung selbst stellt einen diffizilen Implantationsschritt dar. Die zweimalige Durchführung der Sonde durch die Tricuspidalis-Klappe kann deren Funktionsfähigkeit beeinträchtigen. So kann ein hoher Rückstrom des Blutes vom Ventrikel in das Atrium bei der Kontraktion des Ventrikels beobachtet werden. Zudem können durch die doppelt durchgeführte Elektrodensonde die Tricuspidalklappen geschädigt werden.

Ausgehend von der geschilderten Problematik liegt der Erfindung die Aufgabe zugrunde, eine Einzel-Elektrodensonde insbesondere für DDD-Herzschrittmachersysteme zu schaffen, die gegenüber herkömmlichen Sonden einen geringeren Aufwand beim Implantieren und Positionieren hervorruft, andererseits aber eine stark verbesserte und insbesondere sowohl im Atrium als auch Ventrikel wandständige Positionierung der atrialen und ventrikulären Elektroden erlaubt. Zusätzlich sollen Beeinträchtigungen durch eine Mehrzahl von Sondenkörpern sowohl im Blutgefäß als auch innerhalb des Herzens - z.B. die zweimalige Sondendurchführung durch die Tricuspidalklappe - vermieden werden.

Die Lösung dieser Aufgabe ist im Kennzeichnungsteil des Anspruches 1 angegeben. Die demgemäß vorgesehene Erfindung beruht dabei auf der Grundidee, der Sonde auf ihrer im implantierten Zustand im Vorhof plazierten Teillänge eine solche Form zu geben, daß mindestens eine atriale Elektrode - üblicherweise eine Ringelelektrode - der Sonde an der Vorhofwand des Herzens wandständig anlegbar ist. Um dies zu erreichen, ist die Sonde auf der genannten Teillänge mit einem elastischen Vorformelement versehen, das der Sonde dort eine definiert ausgelenkte Form gibt. Aufgrund seiner Elastizität kann das Vorformelement jedoch aus seiner definierten Gestalt in einen im wesentlichen geradlinigen gestreckten Zustand übergeführt werden, was mit Hilfe des üblichen, bei der Sondenimplantation verwendeten Führungsdrahtes erfolgt. Der Führungsdraht muß dabei in seinen Elastizitätseigenschaften natürlich signifikant steifer sein als das Vorformelement.

Beim Implantationsvorgang wird vor der eigentlichen Implantation der Sonde der Führungsdraht bis zum Anschlag an der Sondenspitze vorgeschoben, wobei durch diesen Vorschub des Führungsdrahtes eine Zugkraft auf den Sondenkörper ausgeübt wird, die zu einer Streckung der Sonde im Bereich des Vorformelements führt. In diesem gestreckten Zustand kann dann die Sonde in das Herz eingeführt werden.

Nach der Implantierung der Sonde und dem Herausziehen des Führungsdrahtes wird die Sonde dann auf der besagten Teillänge durch das Vorformelement so ausgelenkt, daß die Sonde mit mindestens einer atrialen Ring-Elektrode an der Vorhofwand des Herzens anliegt.

Da ferner in üblicher Weise die ventrikuläre Elektrode im Bereich der Herzkammer an der Herzkammerwand anliegt, kann eine DDD-Stimulation mit sowohl atrial als auch ventrikulär anliegenden Elektroden mit allen dadurch erzielbaren Vorteilen, wie guten Sensing-Eigenschaften und optimaler Reizschwelle für die Stimulation erfolgen.

In den Ansprüchen 2 und 3 sind vorteilhafte Gestaltungen für das Vorformelement angegeben. Mit diesen Gestaltungen kann erreicht werden, daß mindestens eine atriale Elektrode der Sonde wandständig an der Herzinnenwand anliegt und damit ein guter Kontakt für die Erfassung von Eigenaktionen des Herzens und eine atriale Stimulation hergestellt wird. Insbesondere kann erreicht werden, daß die atriale Elektrode im Bereich der Herzscheidewand - also z.B. an der linken Wand des rechten Vorhofes - positionierbar ist. Die Elektrode liegt damit so weit wie möglich vom Phrenikus-Nerv, der an der rechten Herzaußenseite vorbeiläuft, entfernt, so daß eine als unerwünschter Nebeneffekt auftretende Stimulation des Phrenikus-Nerves, der sich in unmittelbarer Nähe der rechten Wand des Atriums befindet, mit davon hervorgerufenen, unkontrollierten Zwerchfellstimulationen unterbunden werden kann.

Durch die im Anspruch 2 angegebene helixförmige Gestalt des Vorformelements wird eine gute Lagesicherung der Sonde im Vorhof erreicht. Die im Anspruch 3 angegebene omegaförmige Gestalt des Vorformelements ist in diesem Zusammenhang zwar weniger wirksam, jedoch ist der herstellungstechnische Aufwand für dieses Vorformelement geringer. Welcher Variante hier der Vorzug zu geben ist, wird sich nach individuellen Voraussetzungen beim jeweiligen Patienten richten.

Das nach Anspruch 4 vorgesehene weitere Vorformelement auf einer im Ventrikel zu plazierenden Teillänge der Sonde führt dazu, daß nach dem Entfernen des Führungsdrahtes der Elektrodenkopf z.B. eine an sich bekannte J-Form annimmt. Die Elektrode an der Sondenspitze kann damit in der Nähe des AV-Knotens des Herzens positioniert werden, was hinsichtlich einer möglichst optimalen Nachahmung der natürlichen Stimulationsprozesse der Herzmuskulatur vorteilhaft ist. Die vom Herzschrittmacher erzeugten Reize laufen dabei nämlich - analog dem natürlichen Vorgang - ausgehend vom AV-Knoten das Septum hin ab und von dort in die außenseitigen Herzwände.

Ferner ist bei dieser Ausführungsform von Vorteil, daß einerseits beim Implantieren der Sonde diese sich komplett in einem relativ geradlinig gestreckten Zustand befindet, was das Implantieren wesentlich erleichtert.

Beim allmählichen Herausziehen des Führungsdrahtes nach dem Positionieren der Sonde nimmt erst der Elektrodenkopf seine J-Form an, d.h., daß die Elektrodenspitze in eine Richtung weist, die der Auszugsrichtung der Sonde entgegengesetzt gerichtet ist. Dies führt zu einer guten Festlegung der Elektrode an der Sondenspitze im Bereich des AV-Knotens. Diese Festlegung unterstützt ferner die optimale Positionierung der Sonde. Beim weiteren Herausziehen des Führungsdrahtes und der Auslenkung der Sonde im Bereich des Vorhofes aufgrund des dort sitzenden Vorformelementes findet praktisch eine Verkürzung der Sonde statt, die dazu führen könnte, daß die Sonde wieder teilweise aus dem Ventrikel herausgezogen wird. Diesem Vorgang wirkt die besagte Festlegung der Sondenspitze im Bereich des AV-Knotens entgegen.

Die Ansprüche 5 bis 9 kennzeichnen unterschiedliche Ausgestaltungen für die erfindungsgemäß vorgesehenen Vorformelemente. Letztere können beispielsweise aus einem auf der Sonde sitzenden Manschettenteil aus isolierendem Material bestehen, das vorzugsweise aufgeschrumpft wird. Eine andere Alternative besteht darin, das Vorformelement als in den Sondenkörper eingebettete Drahtwendel auszuführen, deren Material z.B. aus einer Memory-Legierung bestehen kann. Es handelt sich dabei um ein Material, das ein "Formgedächtnis" aufweist. So kann die aus der Memory-Legierung bestehende Drahtwendel bei Zimmertemperatur mehr oder weniger stark gestreckt sein, was ein problemloses Einführen der Sonde mit Hilfe des Führungsdrahtes unterstützt. Nach dem Einsetzen der Sonde ist diese der Körpertemperatur ausgesetzt, bei der die Drahtwendel aufgrund ihres "Formgedächtnisses" die entsprechend gewünschte, aus dem Streckzustand ausgelenkte Form annehmen kann.

Eine weitere Alternative für die Ausbildung des Vorformelementes besteht darin, die in der Regel wendelförmigen Zuleitungen für die Elektroden selbst in integraler Weise vorzuformen (Anspruch 9). Hierzu wird die Zuleitung - in der Regel ein Wendeldraht aus medizinischem Stahl MP35N - auf ein formgebendes Wolfram-Werkzeug in Form eines gekrümmten Führungsdrahtes aufgeschoben und dann in geeigneter Weise thermisch behandelt.

Weiterhin ist in einer bevorzugten Ausführungsform der erfindungsgemäßen Einzel-Elektrodensonden vorgesehen, daß die mindestens eine atriale Elektrode mit einer Beschichtung zur Beschleunigung des Einwachsens der Elektrode in die Atrialwand versehen ist. Damit wird in schnellerer Weise das Problem bekämpft, daß die durch die elastische Sondenverformung mechanisch fixierten Ringelektroden auf der atrialen Herzwand wandern und somit eine konstante Reizschwelle für die Stimulation nicht gewährleistet ist. Diese kurz nach dem Einsetzen der Sonde auftretende Schwierigkeit wird erst durch das folgende Einwachsen der Ringelektroden in die Atrialwand des Herzens behoben, was durch die vorgesehene Beschichtung eben beschleunigt werden kann.

Weitere Merkmale, Einzelheiten und Vorteile der Erfindung ergeben sich aus der nachfolgenden Beschreibung, in der Ausführungsbeispiele des Erfindungsgegenstandes anhand der beigefügten Zeichnungen näher erläutert werden. Es zeigen
- Fig. 1A bis C: schematische Schnittdarstellungen des Herzens mit einer Einzel-Elektrodensonde in aufeinanderfolgenden Positionierschritten,
- Fig. 2: einen schematischen, ausschnittsweisen Längsschnitt einer Elektrode in deren atrial zu plazierenden Teil,
- Fig. 3: einen schematischen, ausschnittsweisen Längsschnitt der Elektrode im Bereich deren ventrikulär zu plazierenden Elektrodenkopfs,
- Fig. 4: einen schematischen Querschnitt durch eine Elektroden sonde in einer weiteren Ausführungsform und
- Fig. 5: einen schematischen Schnitt des Herzens mit einer pla zierten Elektrodensonde in einer weiteren alternativen Ausgestaltung.

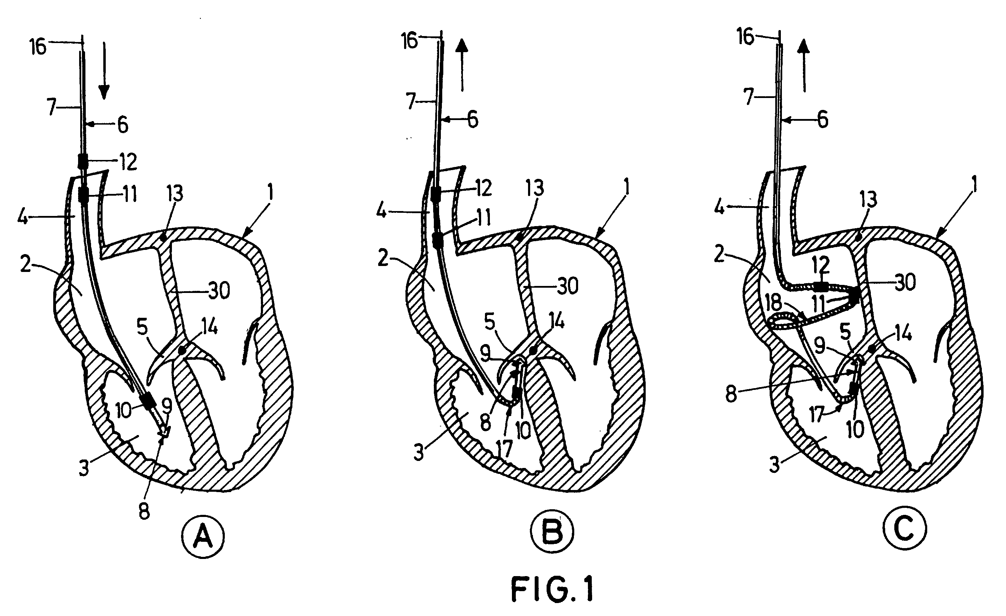

In den Einzeldarstellungen der Fig. 1 ist jeweils das menschliche Herz 1 mit seinem rechten Atrium 2 und seinem rechten Ventrikel 3 sowie der Vene 4 und der Tricuspidalklappe 5 dargestellt.

Eine in Fig. 1 als Ganzes mit 6 bezeichnete Einzel-Elektrodensonde für DDD-Herzschrittmachersysteme weist einen schlauchartigen, flexiblen Sondenkörper 7 aus isolierendem Silikonmaterial auf. An der Spitze 8' des Elektrodenkopfes 8 ist eine erste ventrikuläre Elektrode 9 angeordnet. Ca. 2 bis 3 cm hinter dem Elektrodenkopf 8 sitzt auf dem Sondenkörper 7 eine ventrikuläre Ringelektrode 10, die zusammen mit der ventrikulären Elektrode 9 an der Elektrodenspitze 8' für eine bipolare Stimulation des Ventrikels sorgen kann.

Etwa 10 bis 15 cm hinter dem Elektrodenkopf 8 sitzt ein zweites Elektrodenpaar, bestehend aus den beiden atrialen Ringelektroden 11, 12, die für eine bipolare Stimulierung des Atriums sorgen. Über die Elektroden 9 bis 12 ist im übrigen auch in üblicher Weise ein Sensing der elektrischen Signale im Herzmuskelgewebe möglich, wie sie durch die vom Sinusknoten 13 herrührenden und über den AV-Knoten 14 verzögert zum Ventrikel weitergeleiteten Eigenreize des Herzens hervorgerufen werden.

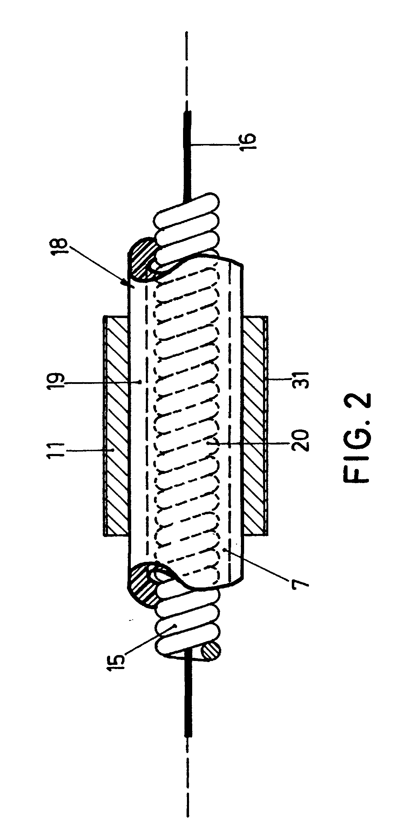

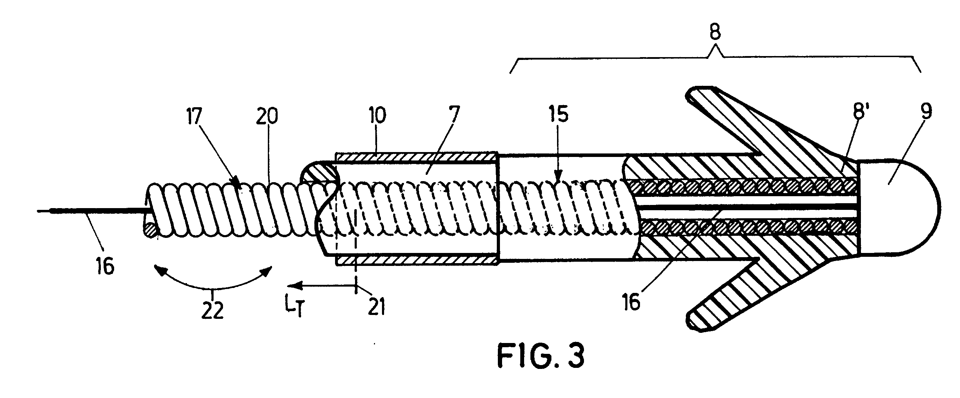

Die Elektroden 9 bis 12 sind ferner in üblicher Weise über isoliert im Sondenkörper 7 geführte elektrische Zuleitungen 15 mit dem eigentlichen Herzschrittmachersystem (nicht dargestellt) verbunden. Von diesen elektrischen Zuleitungen 15 ist in den Fig. 2 und 3 der Übersichtlichkeit halber jeweils nur eine dieser Draht-Zuleitungen 15 gezeigt, die in der Regel wendelförmig angelegt sind. Diese Drahtwendeln bestehen meist aus einem Legierungswerkstoff der Bezeichnung MP35N.

Wie in Fig. 1 ferner schematisch angedeutet ist, weist der Sondenkörper 7 ein zentrales Lumen auf, in das ein elastischer, jedoch relativ steif ausgelegter Führungsdraht 16 bis zur Elektrodenspitze 8' in den Sondenkörper 7 einschiebbar ist.

Mit Hilfe des vollständig eingeschobenen Führungsdrahtes 16, wie in Fig. 1A gezeigt, wird die Einzel-Elektrodensonde 6 durch einen entsprechenden Schnitt in der Vene 4 in im wesentlichen gestreckten Zustand zuerst in das rechte Atrium 2 und dann weiter über die Tricuspidalklappe 5 in das rechte Ventrikel 3 eingeschoben. Dieser Zustand ist in Fig. 1A dargestellt.

Anschließend wird der Führungsdraht 16 einige Zentimeter aus dem Sondenkörper 7 herausgezogen, so daß im Bereich des Elektrodenkopfes 8 die Streckfunktion des Führungsdrahtes 16 entfällt. Demzufolge kann ein kurz hinter der Ringelektrode 10 angeordnetes, in Fig. 1B und C schraffiert angedeutetes Vorformelement 17 zur Wirkung kommen, dessen genaue Ausgestaltungsmöglichkeiten noch anhand der Fig. 2 bis 4 näher zu erläutern sind.

Das Vorformelement 17 weist eine gebogene Grundform auf, so daß der Sondenkörper 7 zum Elektrodenkopf 8 hin den in Fig. 1B und C gezeigten J-förmigen Verlauf einnimmt. Demzufolge weist der Elektrodenkopf 8 schräg nach oben in Richtung des AV-Knotens und die Elektrode 9 kann in dessen direkter Nachbarschaft festgelegt werden.

Wird der Führungsdraht 16 weiter über die atrialen Ringelektroden 11, 12 hinaus aus dem Sondenkörper 7 herausgezogen (Fig. 1C), so entfaltet ein zweites Vorformelement 18 seine Wirkung, das auf einer Teillänge von mehreren Zentimetern im atrialen Bereich des Sondenkörpers 7 angeordnet ist. Das Vorformelement 18 führt zu der in Fig. 1C dargestellten helixförmigen Ausgestaltung des Sondenkörpers 7 im rechten Atrium 2, wodurch sich der Sondenkörper 7 dort an die atriale Herzwand anlegen kann. Insofern liegt die atriale Ringelektrode 11 an der linken, innenseitigen Atrialwand unterhalb des Sinusknotens 13 an, wodurch die Sensing- und Stimulationseigenschaften der Elektrodensonde 6 gegenüber einer flottierenden Elektrode optimiert werden können.

Aufgrund des durch das Vorformelement 18 gegebenen Verlaufs des Sondenkörpers 7 im Atrium ist eine dauerhafte Positionierung des Sondenkörpers 7 in einer optimalen Position möglich, die auch nach dem vollständigen Herausziehen des Führungsdrahtes 16 zuverlässig aufrechterhalten wird.

Es wird im übrigen eine dauerhafte Verankerung der Ringelektroden 11, 12 im Atrium 2 durch deren Einwachsen in die Atrialwand 30 erreicht, was mit der Zeit erfolgt. Um diesen Einwachsprozeß zu beschleunigen, sind nun die Ringelektroden 11, 12 - wie in Fig. 2 anhand der Elektrode 11 angedeutet ist - mit einer Beschichtung 31 versehen, die Collagen-Fasern aufweist.

In dieser Fig. 2 ist eine mögliche Ausgestaltung für das Vorformelement 18 in extrem vergrößerter Darstellung gezeigt. Es handelt sich dabei um ein auf dem Sondenkörper 7 sitzendes Manschettenteil 19 aus isolierendem Silikon-Material, das in seinen Elastizitätseigenschaften steifer als der eigentliche Sondenkörper 7 und weniger steif als der Führungsdraht eingestellt ist. Das Manschettenteil 19 kann im angegebenen Bereich auf den Sondenkörper 7 aufgeschrumpft werden. Es erteilt dem Sondenkörper 7 die in Fig. 1C dargestellte Helixform.

Wie Fig. 2 ferner zu entnehmen ist, ist auf das Manschettenteil 19 die atriale Ringelektrode 11 aufgesetzt, die - wie nicht gezeigt ist - mit einer elektrischen Zuleitung versehen ist. In Fig. 2 ist lediglich eine elektrische Zuleitung 15 in Form eines gewendelten Drahtes dargestellt, der zur ventrikulären Elektrode 9 an der Elektrodenspitze 8 führt. In Fig. 2 ist ferner das Manschettenteil 19 in seiner aufgrund des in das Lumen 23 eingeführten Führungsdrahtes 16 geradlinig gestreckten Form gezeigt.

Fig. 3 zeigt den Elektrodenkopf 8 mit der ventrikulären Elektrode 9 an der Spitze 8' und der dahinter angeordneten Ringelektrode 10. Das hinter der Ringelektrode 10 angeordnete Vorformelement 17 ist im gezeigten Beispiel in integraler Weise durch eine Vorformung der die elektrische Zuleitung für die ventrikuläre Elektrode 9 bildenden Drahtwendel 20 selbst gebildet. Dazu ist auf der Teillänge L_{T}, von der in Fig. 3 lediglich der rechte Rand 21 gezeigt ist, die Drahtwendel 20 so gewickelt, daß sie in einem nicht beaufschlagten Zustand einen durch den Doppelpfeil 22 angedeuteten gebogenen Verlauf einnimmt. Dadurch wird die Biegung am Fuß der J-Form des Elektrodenkopfes 8 erzeugt. In Fig. 3 ist hingegen die Strecklage des Elektrodenkopfes 8 gezeigt, die durch den bis zur ventrikulären Elektrode 9 eingeschobenen Führungsdraht 16 hervorgerufen wird.

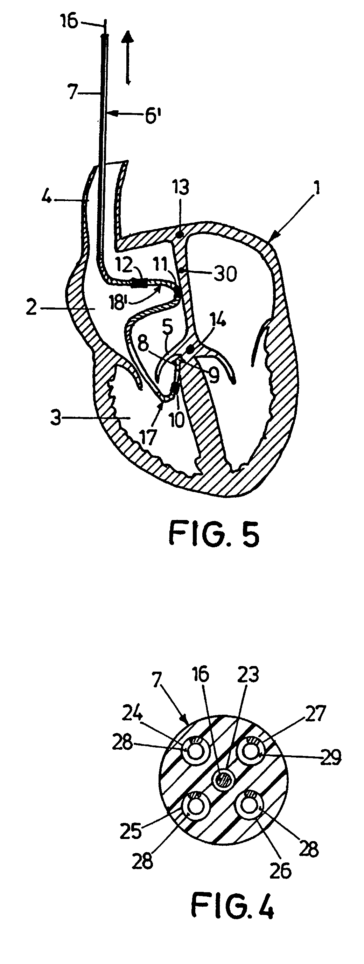

In Fig. 4 ist eine weitere Ausführungsform für eine erfindungsgemäße Elektrodensonde im Schnitt dargestellt. Der aus isolierendem Silikonmaterial bestehende Sondenkörper 7 weist dabei fünf Lumen 23 bis 27 auf, wobei im zentralen Lumen 23 der Führungsdraht 16 sitzt. In den drei weiteren, außenliegenden Lumen 24, 25 und 26 verlaufen wendelförmig gebogene Zuleitungsdrähte 28 beispielsweise für eine ventrikuläre Elektrode an der Elektrodenspitze und eine ventrikuläre Ringelektrode sowie für eine atriale Ringelektrode. Diese Zuleitungsdrähte können auch sogenannte "DFT-Seile" sein, die aus einem elektrisch gut leitfähigen Silberkern und einer diesen umgebenden, mechanisch stabilen Wendel aus Medizinstahl MP35N bestehen.

Im verbleibenden Lumen 27 können Drahtwendeln 29 als Vorformelemente eingesetzt werden, die sich jeweils nur über die Teillängen im Atrium- bzw. Ventrikelbereich der Elektrodensonde 6 erstrecken, um eine dauerhafte Auslenkung aus der Strecklage z.B. entsprechend dem in Fig. 1C gezeigten Sondenverlauf zu erzielen.

Die in Fig. 5 gezeigte Ausführungsform einer erfindungsgemäßen Elektrodensonde 6' weist statt des helixförmigen Vorformelementes 18 ein omegaförmiges Vorformelement 18' auf, so daß die Elektrodensonde eine im wesentlichen in einer Ebene ausgelenkte omegaförmige Gestalt im Bereich des Atriums aufweist. Das Vorformelement 18' kann dabei entweder in Form eines Manschettenteils oder in integraler Weise durch eine entsprechende Formgebung der die elektrische Kontaktierung z.B. für die atriale Ringelektrode 11 bildenden Drahtwendel ausgestaltet sein.

## Patentansprüche

1. Einzel-Elektrodensonde für Herzschrittmachersysteme, insbesondere für DDD-Herzschrittmachersysteme, mit:
- einem schlauchartigen, flexiblen Sondenkörper (7) aus isolierendem Material,
- mindestens einer ventrikulären Elektrode (9, 10) am Sondenkörper (7),
- mindestens einer atrialen Elektrode (11, 12) am Sondenkörper (7),
- isoliert im Sondenkörper (7) geführten elektrischen Zuleitungen (15) für die Elektroden (9, 10, 11, 12) und
- einem Kanal (27) zur Aufnahme eines Führungsdrahtes (16), mit dessen Hilfe die Sonde (6, 6') in das Herz (1) einführbar ist, wobei die Sonde (6, 6') zumindest auf einer im Atrium (2) des Herzens (1) zu plazierenden Teillänge derart mit einem Vorformelement (18, 18') versehen ist, daß bei eingeschobenem Führungsdraht (16) die Sonde (6, 6') im wesentlichen geradlinig gestreckt und bei herausgezogenem Führungsdraht (16) die Sonde auf der Teillänge eine aus der Streckrichtung ausgelenkte, durch das Vorformelement (18, 18') definierte Form annimmt, so daß die Sonde mit mindestens einer atrialen Elektrode (11, 12) an die Atrialwand (30) des Herzens (1) anlegbar ist, **dadurch gekennzeichnet daß** das Vorformelement (17, 18, 18') ein auf dem Sondenkörper (7) auf einer Teillänge sitzendes Manschettenteil (19) aus isolierendem Material ist.

2. Einzel-Elektrodensonde nach Anspruch 1, **dadurch gekennzeichnet, daß** das Vorformelement (18) eine im wesentlichen helixförmig ausgelenkte Gestalt aufweist.

3. Einzel-Elektrodensonde nach Anspruch 1, **dadurch gekennzeichnet, daß** das Vorformelement (18') eine im wesentlichen in einer Ebene ausgelenkte, omegaförmige Gestalt aufweist.

4. Einzel-Elektrodensonde nach einem der Ansprüche 1 bis 3, **dadurch gekennzeichnet, daß** ein weiteres elastisches Vorformelement (17) auf einer im Ventrikel (3) des Herzens (1) zu plazierenden Teillänge (L_{T}) der Sonde derart vorgesehen ist, daß nach dem Entfernen des Führungsdrahtes (16) der Elektrodenkopf (8) eine an sich bekannte Haken-Form annimmt.

5. Einzel-Elektrodensonde nach einem der Ansprüche 1 bis 4, **dadurch gekennzeichnet, daß** das Manschettenteil (19) auf den Sondenkörper (7) aufgeschrumpft ist.

6. Einzel-Elektrondensonde nach einem der Ansprüche 1 bis 5, **dadurch gekennzeichnet, daß** die mindestens eine atriale Elektrode (11, 12) mit einer Beschichtung (31) zur Beschleunigung des Einwachsens der Elektrode (11,12) in die Atrialwand (30) versehen ist.

## Claims

1. A single electrode lead for heart pacemaker systems, in particular for DDD pacemaker systems, comprising:
- a tubular, flexible lead body (7) of insulating material,
- at least one ventricular electrode (9, 10) on the lead body (7),
- at least one atrial electrode (11, 12) on the lead body (7),
- electric lines (15), insulated and guided in the lead body (7), for the electrodes (9, 10, 11, 12), and
- a channel (27) for the accommodation of a guide wire (16), by the aid of which the lead (6, 6') is insertable into the heart (1), wherein at least over a partial length to be placed in the atrium (2) of the heart (1), the lead (6, 6') is provided with a preform element (18, 18') in such a way that with the guide wire (16) pushed in, the lead (6, 6') is substantially straightened and with the guide wire (16) pulled out, the lead, over the partial length, takes a shape deflected from the straightened direction and defined by the preform element (18, 18') so that the lead is placeable on the atrial wall (30) of the heart (1) by at least one atrial electrode (11, 12),
characterized in that the preform element (17, 18, 18') is a cuff member (19) of insulating material placed, over a partial length, on the lead body (7).

2. A single electrode lead according to claim 1, **characterized in that** the preform element (18) exhibits a substantially helically deflected shape.

3. A single electrode lead according to claim 1, **characterized in that** the preform element (18') exhibits an omega-shaped, substantially single-plane deflected configuration.

4. A single electrode lead according to one of claims 1 to 3, **characterized in that** another elastic preform element (17) is provided on such partial length (L_{T}) of the lead as is to be placed in the ventricle (3) of the heart (1) in such a way that after the removal of the guide wire (16), the electrode head (8) takes a shape of a hook known per se.

5. A single electrode lead according to one of claims 1 to 4, **characterized in that** the cuff member (19) is shrunk on the lead body (7).

6. A single electrode lead according to one of claims 1 to 5, **characterized in that** the at least one atrial electrode (11, 12) is provided with a coating (31) to accelerate of the electrode (11, 12) growing in the atrial wall (30).

## Revendications

1. Sonde à électrodes individuelles destinée à des systèmes de stimulation cardiaque, notamment à des systèmes de stimulation cardiaque DDD, comportant :
- un corps (7) de sonde flexible de type tuyau en matériau isolant,
- au moins une électrode ventriculaire (9, 10) sur le corps (7) de sonde,
- au moins une électrode atriale (11, 12) sur le corps (7) de sonde,
- des lignes d'alimentation électrique (15) pour les électrodes (9, 10, 11, 12), disposées de façon isolée dans le corps (7) de sonde, et
- un canal (27) pour la réception d'un fil métallique de guidage (16), à l'aide duquel la sonde (6, 6') peut être introduite dans le coeur (1),
la sonde (6, 6') étant munie d'un élément préformé (18, 18'), au moins sur une longueur partielle à placer dans l'atrium (2) du coeur (1) de telle sorte que, lorsque le fil métallique de guidage (16) est inséré, la sonde (6, 6') soit allongée de façon sensiblement rectiligne et que, lorsque le fil métallique de guidage (16) est retiré, la sonde prenne sur la longueur partielle une forme déviée de la direction allongée, définie par l'élément préformé (18, 18'), de sorte que la sonde puisse être appliquée avec au moins une électrode atriale (11, 12) contre la paroi atriale (30) du coeur (1), **caractérisée en ce que** l'élément préformé (17, 18, 18') est un élément de manchette (19) en matériau isolant disposé sur une longueur partielle du corps (7) de sonde.

2. Sonde à électrodes individuelles selon la revendication 1, **caractérisée en ce que** l'élément préformé (18) présente une forme déviée sensiblement hélicoïdale.

3. Sonde à électrodes individuelles selon la revendication 1, **caractérisée en ce que** l'élément préformé (18') présente une forme d'oméga déviée sensiblement dans un plan.

4. Sonde à électrodes individuelles selon l'une quelconque des revendications 1 à 3, **caractérisée en ce qu**'un autre élément préformé (17) élastique est prévu sur une longueur partielle (L_{T}) de la sonde à placer dans le ventricule (3) du coeur (1) de telle sorte que, après le retrait du fil métallique de guidage (16), la tête (8) d'électrode prenne une forme de crochet en soi connue.

5. Sonde à électrodes individuelles selon l'une quelconque des revendications 1 à 4, **caractérisée en ce que** l'élément de manchette (19) est emmanché par frettage sur le corps (7) de sonde.

6. Sonde à électrodes individuelles selon l'une quelconque des revendications 1 à 5, **caractérisée en ce que** l'électrode atriale (11, 12) au moins est munie d'un revêtement (31) destiné à accélérer l'incarnation de l'électrode (11, 12) dans la paroi atriale (30).
